Europäisches Patentamt

European Patent Office

Office européen des brevets

(11) Publication number: **0 322 394**
**A2**

# (12) EUROPEAN PATENT APPLICATION

(21) Application number: 88870175.2

(22) Date of filing: 23.11.88

(51) Int. Cl.⁴: **C 12 N 15/00**
C 12 P 21/02, A 61 K 39/21
//(C12N15/00,C12R1:19,1:865)

(30) Priority: 24.11.87 US 124198

(43) Date of publication of application:
28.06.89 Bulletin 89/26

(84) Designated Contracting States:
AT BE CH DE ES FR GB GR IT LI LU NL SE

(71) Applicant: SMITHKLINE BIOLOGICALS S.A.
Rue du Tilleul, 13
B-1320 Genval Rixensart (BE)

(72) Inventor: Cabezon, Teresa
Rue de Terheyde, 78
B-1640 Rhode St. Genèse (BE)

De Wilde, Michel
Avenue Gevaert, 136
B-1320 Genval (Rixensart) (BE)

Rutgers, Apolonia
Avenue Coghen, 198
B-1180 Bruxelles (BE)

(74) Representative: Tasset, Gérard
Smithkline Biologicals s.a. Rue de l'Institut 89
B-1330 Rixensart (BE)

(54) Expression of HIV proteins in E. coli and S. cerevisiae.

(57) A recombinant HIV p17 protein expressed in E. coli and
yeast.

EP 0 322 394 A2

Bundesdruckerei Berlin

**Description**

## EXPRESSION OF HIV PROTEINS IN E. COLI AND S. CEREVISIAE

### Field of the Invention

This invention relates to expression of proteins in bacteria and yeast systems. More particularly it relates to the expression of the human immunodeficiency virus (HIV) protein HIV p17 in E. coli and S. cerevisiae.

### Background of the Invention

HIV, the causative agent of AIDS and related disorders, is a member of the Retroviridae family. There exist several isolates of HIV including human T-lymphotropic virus type-III (HTLV- III), the lymphadenopathy virus (LAV) and the AIDS-associated retrovirus (ARV). A related retrovirus, designated HIV type 2, was shown recently to be associated with AIDS in West Africa. Guyader, et al., Nature 326:662(1987).

Molecular characterization of the HIV genome has demonstrated that the virus exhibits the same overall gag-pol-env organization as other retroviruses. Ratner, et al., Nature 313:277(1985). Wain-Hobson, et al., Cell 40:9(1985). In addition, it contains at least five genes that are not found in more ordinary retroviruses : sor, tat3, art/trs, 3′orf and R.

The severity of AIDS makes early and accurate diagnosis of infection by HIV and detection and elimination of HIV-contaminated samples from blood banks extremely important. Gallo, et al., U.S. Patent 4,520,113, disclose use of antigens derived from HTLV-III to detect presence of anti-HTLV-III antibodies in serum. Montagnier, et al., EP-A-138,667, disclose use of a specified LAV antigen to detect infection by the virus. Papas, et al., United States Patent Application Serial No. 6-664,972 (Derwent Accession No. 85-110268/18), disclose expression of a HTLV-I envelope protein coding sequence in E. coli and use of the protein expressed thereby to detect infection by HTLV-1. Crowl, et al., Cell 41:979(1985), report expression in E. coli of portions of the HTLV-III envelope protein gene, env, and use of such proteins for detection of infection by HTLV-III. Casey, et al., J. Virol. 55:417(1985), report purification of one gag gene product, an internal structural protein of HTLV-III referred to as p24, and use of the protein to detect infection by HTLV-III.

Many researchers in the field have been working on the expression in bacteria of the various antigens of the AIDS retrovirus. Recently, novel peptides with sequences of particular fragments of HIV have been constructed for use as reagents for detection of exposure to the AIDS virus. Cosand, U.S. Patent 4,629,783; Capon, et al., EP-A-187041. Use of a recombinant polypeptide from the endonuclease region of the AIDS retrovirus polymerase gene (pol) to detect serum antibodies in infected individuals is described by Steimer, et al., J. Virology 58:9(1986).

A polypeptide expressed by cells transformed with a recombinant vector containing HTLV-III cDNA encoding the env gene sequence and the use of such immunoreactive polypeptide in assays for the detection of HTLV-III is described in Chang, EP-A-185 ,444. Dowbenko, et al. disclose the expression in E. coli of the AIDS retrovirus p24 gag protein and its use as a diagnostic reagent. Synthesis in E. coli of the HTLV-III core antigens and immunoreactivity with human serum is also reported by Ghrayeb, et al., DNA 5 :93(1986) and Steimer, et al., Virology 150:283(1986).

Studies are underway to develop a vaccine against the AIDS virus. A recent report described initial studies carried out in an attempt to define the thymosin $\alpha_1$-immunoreactive protein in the AIDS virus, to determine if a synthetic peptide containing the immunoreactive region was immunogenic and if antibodies to the peptide recognized a region of the HIV p17 protein. Naylor, et al., Proc. Natl. Acad. Sci. USA 84:2951(1987).

Infection of cells by viruses involves viral recognition of cellular surface receptors, followed by a redistribution of virus particles on the cell surface. Certain viruses penetrate the plasma membrane directly but many are endocytosed at coated pits and subsequently pass into a prelysosomal endosome. The low pH environment of the endosome catalyses a fusion reaction between the membrane of the virus particle and that of the prelysosomal endosome thus enabling the viral core to enter the cytoplasm. Patterson, et al., Vaccine 4:79(1986). It has been suggested that HIV-1 is internalized by the receptor-mediated endocytosis pathway. Maddon, et al., Cell 47:333(1986). The initial recognition of HIV of its target cell is by means of the viral surface protein gp120 which binds to the CD4 antigen of the target cell.

In view of the considerable heterogeneity and antigenic variation observed in the gp160/gp120 surface proteins, vaccines based on interference with the initial attachment step in the viral entry process seems not to be an effective approach.

The gag protein p17, however, with its submembrane localization (Gelderblom, et al., Virology 156: 171( 1987) ) is well positioned to be in close con tact with the transmembrane protein gp41 and the viral membrane or alternatively with gag p24 and possibly gag p15 viral RNA thereby playing a central role in the conformational changes involved in the viral entry and uncoating process. Furthermore, gag p17 has been found to have a myristylated N-terminus. Myristylation has been implicated in virion assembly and transport of viral components to the plasma membrane. Rein, et al., Proc. Natl. Acad. Sci. 83:7246(1986), Rhee, et al., J.

Virol. 61:1045(1987). Myristylated proteins are generally localized in the plasma membrane. Wilcox, et al., Biochemistry 26:1029(1987).

The gag p17 protein is an attractive target for immunological studies to establish its HIV neutralizing and protective potential. Direct evidence for the potential of p17 to induce neutralizing antibodies comes from the observation that antiserum against thymosin $\alpha_1$ effectively neutralizes HIV infection. Thymosin $\alpha_1$ contains a region of 18 amino acids that has a 44 to 50% homology to p17. Sarin, et al., Science 232:1135(1986). A synthetic p17 peptide analogue covering the region of homology with thymosin $\alpha_1$ induces antibodies which neutralize several different strains of HIV in vitro. Naylor, et al., Proc. Natl. Acad. Sci. USA 84:2951(1987).

Recently, various hypotheses as to the most effective type of immunogen for use as an AIDS vaccine have been proposed. Salk, Nature 327:473(1987).

## Summary of the Invention

In one embodiment of the invention, an E. coli expression vector having a DNA coding sequence for HIV p17 protein or derivatives of p17 fused in frame to regulatory elements functional in E. coli is described.

In another embodiment of the invention, a yeast expression vector, preferably for use in the yeast strain S. cerevisiae, is described. The vector harbors a DNA coding sequence for HIV p17 protein or derivatives of p17 fused in frame to regulatory elements functional in yeast.

In another embodiment is described a method for producing HIV p17 protein in E. coli which comprises culturing the organism transformed with an HIV p17 expression vector under permissive conditions, such that the protein is expressed in recoverable quantities, and isolating the protein from the culture system.

In another embodiment of the invention, a method of producing HIV p17 protein in yeast which comprises culturing the organism transformed with an HIV p17 expression vector under permissive conditions is described. The protein is expressed in recoverable quantities and can be isolated from the culture system.

Another embodiment of the invention is a vaccine for stimulating protection in individuals against HIV infection which comprises one or more recombinant HIV-antigens and a suitable carrier thereof.

## Detailed Description of the Invention

To prepare an expression vector of the invention, a DNA sequence which codes for p17, or an immunologically equivalent derivative thereof, is fused in frame to regulatory elements which are functional in E. coli or yeast. The p17 coding sequence is prepared by known techniques from HIV or from HIV-infected cells by isolation of viral mRNA and preparing cDNA by reverse transcription.

cDNA clones carrying the p17 coding sequence can be identified by use of oligonucleotide hybridization probes. The probes can be designed based on the known sequence of p17 which is reported (See Ratner, et al., supra). Having identified a clone carrying the p17 coding sequence, the coding sequence can be excised by use of restriction endonucleases and inserted into cloning and/or expression vectors. Alternatively, an HIV p17 expression cassette can be integrated such as through use of the Ty transposons (Boeke, et al., Cell 40:491(1985). By "expression cassette" is meant the p17 coding sequence and regulatory regions necessary for expression. The p17 coding sequence can also be obtained from HIV libraries, e.g., λBH10, as reported by Shaw, et al., Science 226:1165(1984). The coding sequence can also be synthesized by chemical DNA synthetic techniques.

The coding sequence for the HIV p17 submembrane protein can be inserted into an E. coli or yeast expression vector as described in this invention.

In the preferred practice of the invention, the p17 coding sequence is inserted into an E. coli or yeast expression vector so that "authentic" p17 is expressed. By "authentic" is meant the p17 protein being expressed has the complete amino acid sequence for p17 with the N-terminal methionine and no deletions or alterations. In addition the p17 is expressed as a complete protein, not as a fragment of one of the larger HIV proteins, for example p55 gag. Also, the correct insertion of the C-terminal region of the p17 protein of the instant invention into the expression vectors in E. coli or yeast results in direct expression of p17 due to the position of the stop codon. The stop codon is inserted at the point where the complete gag protein is normally processed.

By "regulatory region" is meant the expression control sequence, for example, a promoter and ribosome binding site, required for transcription and subsequent translation. Regulatable regulatory regions, that is, regulatory signals which are not constitutive but require induction or derepression, are preferred. Such vectors typically comprise, in addition to the regulatory element, a region which permits the vector to be stably maintained in a host cell population, that is, a replicon or origin of replication, and one or more selection markers, i.e., genes which confer a selectable phenotype upon hosts carring the vector.

Derivatives of the coding sequence for the HIV-protein can be prepared using standard recombinant DNA or synthetic techniques. In addition to the use of p17 wild type coding sequence, examples include addition, substitution or deletion of one or more base pairs or radiation or chemical mutation such that the resulting protein being expressed while varying from the "native protein" still retains its immunologic properties, i.e., can elicit an immune response which inhibits infection by HIV.

Exemplary expression vectors which may be used in the practice of the invention in E. coli include, for example, pAS1, Rosenberg, et al., Meth. Enzymol. 101:123(1983); pCQV2, Bollen, et al., FEBS Letters 166:67(1984), or the trp promoter, McGrath, et al., Nature 295:423(1982). In yeast, exemplary expression vectors which may be used in the practice of the invention include, for example, vectors based on the PHO5 promoter (Miyanohara, et al., Proc. Natl. Acad. Sci USA 80:1(1983); Kramer, et al., Proc. Natl. Acad. Sci USA 81:367(1984)), the TRP1 promoter (Dobson, et al., Nucleic Acids Research 11:2287(1983)) and the PGK promoter (Mellor, et al., Gene 24:1(1983); Hitzeman, et al., Science 219:620(1983)). In a preferred practice of the invention, the yeast expression vectors, ARG3 and TDH3 are used as more fully described in the Examples.

The HIV p17 protein fragments and derivatives may also be expressed as a fusion protein with, for example, other HIV related proteins, i.e. , p24, p15 or proteins of the env or pol region, the hepatitis B surface antigen (HBsAg), products of the galactokinase (galK) gene or all or a portion of the influenza NS1 protein (Young, et al., Proc. Natl. Acad. Sci. USA 80:6105(1983)).

The recombinant HIV p17 produced in accordance with the invention can be isolated and harvested by culturing the microorganisms or cells which have been transformed with the expression vector of the invention. The culturing conditions, e.g., analytes, metabolites, other medium ingredients and the temperature are selected so as to enhance the expression of the p17 protein. Typically, transformed E. coli cultures are cultured in a nutrient broth containing assimilable sources of carbon and nitrogen, with aeration and under selection pressure, until log phase growth ($A_{620}$ about 0.4-0.6). Prior to induction and then for an additional 1-1/2 to 5 hours following induction, the cultures continue to grow until a recoverable quantity of the protein is expressed.

Transformed yeast cultures typically are cultured in a yeast nitrogen base containing glucose in the absence of amino acids and grown to an $A_{620}$ of 0.4 -0.6. p17 expressed by the yeast cells can be extracted from the culture system, i.e., p17 remains within the cell after expression, using standard cell extraction techniques.

The HIV p17 protein isolated from the culture system can be further purified using standard protein purification techniques, checked for purity and stored for later formulation.

The recombinant proteins produced using the methods of this invention are suitable for use as vaccines in humans. A pharmaceutical composition consisting of the HIV-antigen, for example, the mature gag structural protein p17 alone or in combination with p24 and p15 , along with a suitable carrier or adjuvant may be used to prevent the onset of HIV associated diseases.

An effective HIV vaccine should prevent virus mediated as well as cell-to-cell transmission of infection and thus must be able to elicit both humoral and cell-mediated immunity.

By itself or in association with lipids or in combination with other HIV proteins, p17 can be used in a vaccine for inhibiting initial infection of a person by HIV, for inhibiting infection of cells in a person exposed to the virus and for inhibiting cell to cell spread of the virus. Use of the recombinant p17 protein or its derivatives for production of a vaccine for human use has the added advantage that the preparation is not derived from an attenuated or otherwise inactivated live virus preparation.

In the vaccine of the invention, an aqueous solution of the p17 protein of the invention preferably buffered at physiological pH, can be used directly. Alternatively, the p17 protein, with or without prior lyophilization, can be mixed or adsorbed with any of the various known adjuvants. Such adjuvants include, among others, aluminum hydroxide, muramyl dipeptide and saponins such as Quil A. The p17 protein can also be formulated with known pharmaceutically acceptable carriers, for example, sodium chloride, acetates, glucose, mannitol and albumin. In another alternative, the p17 protein can be encapsulated within microparticles such as liposomes or conjugated to a known immunostimulating macromolecule, such as killed Bordetella or a tetanus toxoid.

Alternatively, a multivalent vaccine comprising p17 and one or more other vaccinal agents can be prepared. Such other agents can be, for example, other HIV-antigens or antigens from a different pathogenic microorganism, virus or cell.

In addition, a vaccine comprising a fused protein, for example, p17 fused to the S protein of hepatitis B virus can be prepared using the E. coli or yeast expression systems of the invention. Using the p17 fusion protein coding sequence as described in Example 6, the fused protein can also be expressed using mammalian cell p17-S protein expression plasmids or vaccinia recombinant virus expressing the p17-S protein. Other fusions can also be made, for example p17 fused with plasmodium CSP repeats. Simultaneous expression of the fusion protein with gp41 or gp160 in yeast, mammalian cells or the vaccinia expression system can also be carried out.

A general description of the methods for vaccine preparation can be found, for example, in New Trends and Developments in Vaccines, edited by Voller, et al., University Park Press, Baltimore. Maryland, U.S.A. (1978); Dalsgaard, et al., Acta. Vet. Scand. 18:349(1977); Likhite, U.S. Patent 4,372,945 and Armor, et al., U.S., Patent 4.474,757.

The amount of p17 protein present in each vaccine in order to comprise an effective amount of vaccine, i.e., an amount which will stimulate an immunoprotective response, will depend on whether the vaccine is adjuvanted. Generally, it is expected that each dose will comprise 0.1 to 1000 μg of p17 protein, preferably 1 to 100 μg. An optimal amount can be determi ned by standard clinical studies wherein antibody titer and other immune responses are monitored. Following an initial vaccination, subjects will preferably be given a boost vaccination, e.g., after 4 weeks, followed by repeat boosts, for example, every six to twelve months.

In addition to use of the p17 protein expressed in E. coli or yeast for production of a vaccine against AIDS, the expressed protein can be used as a diagnostic reagent.

As an immunodiagnostic reagent, the p17 protein can be used in any of the standard immunodetection assays, for example, enzyme linked immunosorbent assay (ELISA), "sandwich immunoassays", competitive binding assays or radioimmune assays (RIA) for the detection of HIV seroconversion.

The p17 protein, derivatives or fragments thereof, may also be used to stimulate production of anti-sera which is reactive with HIV. The ability to raise polyclonal antibodies also renders it possible to produce monoclonal antibodies by the standard techniques originally described by Kohler and Milstein, Nature 256:495(1975) or other techniques of cell fusion or transformation.

Antibodies directed against p17 or fragments of p17 can also be useful in detecting the presence of p17 gene product in a cell culture. In addition, such antibodies are useful, for example, in affinity purification, construction of cDNA probes and as neutralizing agents in therapy for HIV infection.

The p17 protein can also be used as an effective therapeutic agent against HIV. As a therapeutic, p17 can be used to prevent or diminish virus mediated as well as cell to cell transmission of infection. p17 may be used alone or in combination with other agents, for example other HIV proteins or antiviral agents such as azidothymidine (AZT).

## Examples

The examples which follow are illustrative, and not limiting of the invention.

## Example 1

Preparation of the E. coli p17 cloning vector pRIT12920

Using the plasmid pBH10 as starting material, the gag region of HIV was subcloned. By genetic manipulation of this region two intermediate plasmids were obtained: pRIT12915 and pRIT12917. Plasmid pRIT12917 contains a modular DNA fragment corresponding to the coding region for p17. Using methods known to those skilled in the art of genetic engineering (see, for example, "Molecular Cloning", Maniatis, et al., Cold Spring Harbor Laboratory, New York (1982)), the plasmid so constructed was used to express HIV p17 protein in either E. coli or yeast.

The starting material was the clone BH10-R2 which contains the entire cDNA of HIV. BH10-R2 is a derivative of the plasmid pBH10 constructed by cloning the Sst I insert of λBH10 (Shaw, et al., supra) into the Sst I site of pSP64 (Butler, et al., J. Biol. Chem. 257:5772(1982). The cloning strategy included two steps :

a) Subcloning of the gag region from BH10-R2 in order to generate a p17 gene fragment

Digestion of BH10-R2 with the PstI restriction enzyme yielded a PstI fragment that contains the HIV sequence extending from base 679 to base 1416. The PstI fragment was inserted at the PstI site of the vector pS2. pS2 is a pUC18 derivative harboring a DNA insert containing an NcoI linker in a SalI site. The PstI fragment was inserted in such an orientation that the N-terminal p17 region was placed near to the NcoI site. The intermediate plasmid pRIT12915 was thereby obtained. This vector has a ClaI site overlapping the 14th amino acid codon.

pRIT12915 was digested with NcoI and ClaI restriction enzymes and the largest fragment was purified and ligated with an NcoI-ClaI synthetic adaptor harboring the N-terminal portion of the p17 coding sequence. The p17 N-terminal adaptor has the following codon sequence :

```
5' C ATG  GGT  GCT  AGA  GCT  TCC  GTG  TTG  TCC  GGT  GGT  GAA
        TTG  GAT  3'   CCA  CGA  TCT  CGA  AGG  CAC  AAC  AGG
        CCA  CCA  CTT  AAC  CTA  GC

   NcoI extension              ClaI extension
```

The codon sequence was changed in accordance with optimal codon usage in E. coli and yeast, i.e., the codon for specific amino acids was chosen such that an efficient and preferred reading sequence was obtained. The plasmid pRIT12917 so constructed contains an NcoI site overlapping the ATG initiator codon of the p17 N-terminus. An XbaI site overlaps the stop codon that is 33 base pairs downstream from the C-terminal sequence of the mature p17.

### b) Insertion of the p17 coding region into the E. coli expression vector

A 429 bp Ncol Rsal fragment isolated from pRIT12917 was ligated to the pOTS-Ncol E. coli expression plasmid. Plasmid pOTS Ncol is a derivative of plasmid pAS1 described in Rosenberg, et al., Meth. Enzymol. 101:123(1983). pOTS-Ncol utilizes the same promoter and ribosome binding site for translation and transcription as used in pAS1. The BamHI restriction site of pAS1 was changed to an Ncol site overlapping the ATG initiation codon by insertion of a synthetic oligonucleotide resulting in pOTS-Ncol. pOTS-Ncol was previously opened with Xbal restriction enzyme, T₄ polymerase repaired and digested with Ncol restriction enzyme. The largest fragment was ligated to the 429 bp Ncol-Rsal fragment. The E. coli p17 expression plasmid pRIT12920 was thereby obtained and was used to transform to ampicillin resistance two E. coli strains: AR120 and AR58. Mott, et al., Proc. Natl. Acad. Sci. 82:88(1985).

### Example 2

#### Preparation of the E. coli p17 expression plasmid pRIT13017

The plasmid pRIT12920 described in Example 1 was digested with Ncol and Pvull restriction enzymes in order to obtain a 358 bp Ncol-Pvull fragment harboring the N-terminal coding region of p17 protein. A Pvull-Xhol synthetic adaptor was prepared containing the C-terminal coding region of p17 as follows :

```
 PvuII extremity                                       Stop codon
        |                                                   |
5'CT GAC ACT GGT CAC TCT TCT CAA GTT TCT CAA AAC TAC TGA
TCA C 3' GA CTG TGA CCA GTG AGA AGA GTT CAA AGA GTT TTG
ATG ACT AGT G AGC T
             |
         Xhol extension
```

The codon sequence was changed in accordance with optimal codon usage in E. coli and in yeast as described in Example 1. The 358 bp Ncol Pvull fragment and the 45bp Pvull-Xhol synthetic adaptor were ligated to pOTS-Ncol plasmid previously open with Ncol and Xhol enzymes. The E. coli plasmid pRIT13017 that expresses the mature p17 protein in E. coli was thereby obtained.

### Example 3

#### Preparation of the yeast expression vector pRIT12914

The starting material was the yeast expression vector pRIT10774, a 2 μ partial yeast expression vector containing an expression cassette with an unique BamHI site between an ARG3 promoter and transcription termination regions. Construction of plasmid pRIT10774 is described in Cabezon, et al., Proc. Natl. Acad. Sci. USA 81:6594(1986).

Digestion of pRIT10774 with HindIII restriction enzyme gives a HindIII fragment harboring the expression cassette. This fragment was mixed with pRIT12377 (Harford, et al., Curr. Genet. 11:315-319(1987)) which was previously opened with BamHI enzyme. pRIT12377 is a complete 2μ based yeast vector carrying the LEU2 gene thereby providing leucine selection. The mix of fragment and plasmid was treated with Klenow DNA polymerase in order to repair the cohesive ends and ligated. The yeast expression vector pRIT12914 was thereby obtained.

pRIT12914 contains the complete yeast 2μ DNA sequences, the yeast LEU2 gene necessary for selection and maintenance of the plasmid in yeast, the E. coli replicon sequences and E. coli ampicillin gene necessary for the selection and replication of the plasmid in E. coli, and a yeast expression cassette composed of the ARG3 promoter and the ARG3 terminator sequences separated by a unique BamHI.

### Example 4

EP 0 322 394 A2

## Preparation of the yeast p17 expression plasmids pRIT12953 and pRIT12957

Starting with the E. coli p17 expression vector pRIT12920 of Example 1, a 429 bp p17 fragment was generated by digestion with NcoI and XbaI restriction enzymes. This fragment was inserted in two different yeast expression plasmids. The first one, pRIT12914, as described in Example 3, harbors an expression cassette with a single BamHI site between the ARG3 promoter and terminator regions and is useful for expression of gene fragments harboring their own ATG initiator codon. The plasmid pRIT12914 opened with BamHI enzyme and filled in with Klenow DNA polymerase was ligated to the NcoI-XbaI p17 fragment previously filled in with Klenow DNA polymerase fragment, resulting in pRIT12953.

The second yeast expression plasmid, pRIT12544 (Harford, et al., Cure. Genet. 11:315(1987)) is identical to pRIT12914 except for the expression cassette. pRIT12544 harbors an expression cassette with a single BamHI site between a TDH3 promoter and ARG3 terminator regions and is useful for expression of gene fragments devoid of the ATG initiator codon.

pRIT12544 was digested with BamHI restriction enzyme treated with Mung bean nuclease and ligated to the NcoI-XbaI p17 fragment. This fragment was prepared in such a way that the NcoI extension was deleted by Mung bean nuclease digestion and the XbaI extension was filled in with the Klenow DNA polymerase in order to conserve the TAG stop codon. The plasmid pRIT12957 was thereby obtained. pRIT12953 and pRIT12957 were used to transform to LEU+ the yeast strain TCY1 (Hoylaerts, et al., FEBS Letters 204:83(1986)) from which was obtained the yeast p17 expression strains Y684 and Y686.

## Example 5

## Preparation of the yeast p17 expression plasmid pRIT13016

Starting with the E. coli p17 expression vector pRIT13017 as described in Example 2, a 330 bp p17 fragment was generated by digestion with NcoI and XhoI restriction enzymes. This fragment was mixed with the plasmid pRIT12914 (described in example 4) previously open with BamHI restriction enzyme. The mix was filled in with Klenow DNA polymerase and ligated. The yeast plasmid pRIT13016 expressing the mature p17 protein was obtained. pRIT13016 was used to transform to LEU+ the yeast strain TCY1 from which was obtained the yeast p17 expression strains Y734.

## Example 6

## Construction of an expression vector for a p17-S fusion protein

The construction of an expression vector for HIV p17-S protein of hepatitis B virus fusion protein can be carried out in two steps :

### a) Construction of an intermediate plasmid containing the coding sequence for a p17-S fusion protein

The starting materials are the E. coli expression plasmid pRIT13017 (described in Example 2) and the plasmid pRIT10777. Plasmid pRIT10777 is a pBR322 derivative containing the S gene flanked by a XhoI site adjacent to the second codon and a BamHI site adjacent and downstream of the stop codon. The SalI site of pBR322 is placed 281 bp downstream of the stop codon.

The plasmid pRIT13017 harboring the p17 coding region flanked with an NcoI site (overlapping the ATG codon), a BclI site (overlapping the stop codon) and a XhoI site (beyond the stop codon) can be opened with XhoI restriction enzyme and ligated to the Xho-SalI S fragment derived from pRIT10777.

A resulting plasmid which retains the XhoI site has the S gene in the correct orientation adjacent and downstream of the p17 coding sequences. In order to eliminate the stop codon at the end of the p17 gene and obtain the correct reading frame for the expression of a p17-S fusion protein, the intermediate plasmid can be opened by BclI and XhoI digestion, mung bean nuclease treated to digest the unwanted extensions and religated. The resulting plasmid contains the p17 coding region in frame with the S coding region.

### b) Construction of a yeast expression vector for a p17-S fusion protein

An NcoI-BamHI fragment can be prepared from the plasmid harboring the coding region for the fusion p17-S protein and inserted into the BamHI site of pRIT12914 (described in Example 3). After filling in with Klenow DNA polymerase and ligation, a p17-S yeast expression vector is obtained.

## Example 7

## Characterization of the E. coli and yeast product

E. coli strains harboring one of plasmid pRIT12920, pRIT13017 or pOTS-NcoI were grown and induced as described by Mott, et al., Proc. Natl. Acad. Sci. USA 82:88(1985). Yeast cells harboring plasmid pRIT12953,

7

pRIT12957, PRIT12914, PRIT12544 or pRIT13016 were grown in Difco yeast nitrogen base without amino acids 0.675%, containing 2% glucose into log phase, and collected by centrifugation. To the packed cells (0.1 gram), first 20 µl of 40 mM PMSF (phenylmethylsulfonylfluoride) in isopropanol, and then 200 µl of sample buffer for SDS-polyacrylamide gel electrophoresis (0.125 M tris-HCl, PH 6.8 containing 20% glycerol, 4% SDS, 6 M urea and 10% 2-mercapto-ethanol), were added. Samples were vortex mixed and aliquots of 2 to 20 µl were further diluted in sample buffer to a final volume of 40 µl, incubated for 5 minutes at 100° C and electrophoresed through a 15% separating, 5% stacking gel according to the method of Laemmli, Nature 227:680(1971).

Synthesis of p17 related protein was detected by "in situ" immunedetection with specific antibodies (See Burnette, Anal. Biochem. 112:195(1981); Gershoni, et al., Anal. Biochem. 131:1(1983) Towbin,et al., J. Immunol. Methods 72:313 ( 1984) ). A p17 related protein was detected among the total proteins of E. coli strains harboring plasmids pRIT12920 or pRIT13017 and the yeast strains harboring plasmids pRIT12953, pRIT12957 or pRIT13016, while no p17 related protein was detected among the total proteins of E. coli strains harboring pOTS-NcoI and yeast strains harboring pRIT12914 or pRIT12544.

Cells from yeast strain TCY1 harboring pRIT12914, pRIT12953, pRIT12544, pRIT12957 or pRIT13016 were grown in Difco yeast nitrogen base without amino acids 0.675%, containing 2% glucose to an $OD_{620\ nm}$ of 0.4. Cells (20 ml culture) were labeled for 60 min. with 1 mCi of $^3$H labeled myristic acid (New England Nuclear, Boston, Massachusetts) and then collected by centrifugation. Cells were washed with cold $H_2O$, resuspended in 100 µl 5 mM tris-HCl (pH 7.4) containing 3 mM dithiothreitol (DTT), 1% sodium dodecyl sulfate (SDS) and 1 mM PMSF, and broken with 100 mg glass beads by six 30-second spurts of vigorous mixing in a Vortex mixer, cooling on ice between each mixing. Debris was removed by centrifugation for 1 minute in an Eppendorf 5414 centrifuge. Towler, et al., Proc. Natl. Acad. Sci. 83:2812(1986). 20 µl of the extract was treated for 3.5 hours at room temperature with 7 µl of freshly prepared 4M hydroxylamine, 20 mM glycine, pH 10.

Hydroxylamine treated and untreated samples containing $10^5$ cpm were subjected to SDS polyacrylamide gel electrophoresis and fluorography. After electrophoresis, gels were fixed in 40% methanol 10% acetic acid, treated with Amplify® (Amersham International, United Kingdom) for fluorography, dried on filter paper under vacuum and subjected to fluorography using Kodak-X-Omat® S film at -70°.

Results indicated that pRIT12953, pRIT12957 and pRIT13016 containing cells synthesize a protein of approximately 17kd, which is hydroxylamine stable, indicating the $^3$H-myristate label is present in an amide linkage. No labeled bands specific for the extracts from cells harboring pRIT12914 or pRIT12544 were detected.

The yeast product was found to contain covalently bound myristic acid present in an amide linkage. Thus, the yeast product is a post-translationally modified protein that carries a myristoyl fatty acid residue attached to the N-terminal glycine as has been found to be the case for the viral p17. (Sarngadharan, et al., Communication 125, Abstracts of Intl. Conf. on AIDS, June 1986, Paris, France).

The above description and examples fully disclose the invention including preferred embodiments thereof. Modifications of the methods described that are obvious to those of ordinary skill in molecular genetics and related sciences are intended to be within the scope of the following claims.

## Claims

1. An E. coli expression vector having a DNA coding sequence for HIV p17 or derivatives of p17 fused in frame to regulatory elements functional in E. coli.

2. The expression vector of claim 1 having regulatory elements of the PL promoter of lambda.

3. The expression vector of claim 2 having the regulatory elements of pAS1.

4. The expression vector of claim 1 having the coding sequence for mature HIV p17 protein.

5. The expression vector of claim 4 having regulatory elements of the PL promoter of lambda.

6 . The expression vector of claim 5 having the regulatory elements of pAS1.

7. The expression vector of claim 6 having a DNA coding sequence for p17 which is plasmid pRIT13017 or a derivative thereof.

8. HIV p17 expressed by E. coli transformed with the vector of claim 1.

9. The expression vector of claim 4 wherein the p17 N-terminal coding region is as shown below :

```
5'  C ATG  GGT  GCT  AGA  GCT  TCC  GTG  TTG  TCC  GGT  GGT  GAA

        TTG  GAT  3'  CCA  CGA  TCT  CGA  AGG  CAC  AAC  AGG

        CCA  CCA  CTT  AAC  CTA  GC
```

10. The expression vector of claim 4 wherein the p17 C-terminal coding region is as shown below :

5′ CT GAC ACT GGT CAC TCT TCT CAA GTT TCT CAA AAC TAC TGA TCA C 3′ GA CTG TGA CCA GTG
AGA AGA GTT CAA AGA GTT TTG ATG ACT AGT G AGC T

11. The expression vector of claim 9 wherein the p17 C-terminal coding region is as shown below :

5′ CT GAC ACT GGT CAC TCT TCT CAA GTT TCT CAA AAC TAC TGA TCA C 3′ GA CTG TGA CCA GTG
AGA AGA GTT CAA AGA GTT TTG ATG ACT AGT G AGC T

12. The expression vector of claim 5 wherein the p17 N-terminal coding region is as shown below :

```
5'  C ATG  GGT  GCT  AGA  GCT  TCC  GTG  TTG  TCC  GGT  GGT  GAA

         TTG  GAT   3'  CCA  CGA  TCT  CGA  AGG  CAC  AAC  AGG

         CCA  CCA  CTT  AAC  CTA  GC
```

13. The expression vector of claim 12 wherein the p17 C-terminal coding region is as shown below :

5′ CT GAC ACT GGT CAC TCT TCT CAA GTT TCT CAA AAC TAC TGA TCA C 3′ GA CTG TGA CCA GTG
AGA AGA GTT CAA AGA GTT TTG ATG ACT AGT G AGC T

14. An E. coli cell transformed with the vector of claim 1.

15. The expression vector of claim 1 having the coding sequence for mature p17 protein linked with a portion of the coding sequence for p24 protein.

16. The expression vector of claim 15 which is pRIT12920 or a derivative thereof.

17. A yeast expression vector having a DNA coding sequence for HIV p17 or derivatives of p17 fused in frame to regulatory elements functional in yeast.

18. The expression vector of claim 17 having a promoter selected from the group : ARG3, TDH3, PHO5, TRP1 and PGK.

19. The expression vector of claim 17 having a p17 coding sequence operatively linked with ARG3 promoter and termination sequences.

20. The expression vector of claim 17 having a p17 coding sequence operatively linked with a TDH3 promoter and termination sequences.

21. The expression vector of claim 17 having the coding sequence for mature HIV p17.

22. The expression vector of claim 21 which is pRIT13016 or a derivative thereof.

23. The expression vector of claim 21 wherein the p17 N-terminal coding region is as shown below :

```
5'  C ATG  GGT  GCT  AGA  GCT  TCC  GTG  TTG  TCC  GGT  GGT  GAA

         TTG  GAT   3'  CCA  CGA  TCT  CGA  AGG  CAC  AAC  AGG

         CCA  CCA  CTT  AAC  CTA  GC
```

24. The expression vector of claim 23 wherein the p17 C-terminal coding region is as shown below :

5′ CT GAC ACT GGT CAC TCT TCT CAA GTT TCT CAA AAC TAC TGA TCA C 3′ GA CTG TGA CCA GTG
AGA AGA GTT CAA AGA GTT TTG ATG ACT AGT G AGC T

25. The expression vector of claim 21 wherein the p17 C -terminal coding region is as shown below :

5′ CT GAC ACT GGT CAC TCT TCT CAA GTT TCT CAA AAC TAC TGA TCA C 3′ GA CTG TGA CCA GTG
AGA AGA GTT CAA AGA GTT TTG ATG ACT AGT G AGC T

26. HIV p17 expressed by a yeast cell transformed with the expression vector of claim 17.

27. A yeast cell transformed with the vector of claim 17.

28. The yeast cell of claim 27 which is the strain S. cerevisiae.

29. The yeast cell of claim 28 which is the S. cerevisiae TCY1 strain.

30. The expression vector of claim 17 having the coding sequence for mature p17 protein linked with a portion of the coding sequence for p24 protein.

31. The expression vector of claim 30 which is pRIT12953 or a derivative thereof.

32. The expression vector of claim 30 which is pRIT12957 or a derivative thereof.

33. A method of producing HIV p17 protein in E. coli which comprises culturing the organism transformed with an HIV p17 expression vector under permissive conditions, such that the protein is expressed in recoverable quantities, and isolating the protein from the culture system.

34. The method of claim 33 wherein the expression vector is plasmid pRIT13017 or a derivative thereof.

35. The method of claim 33 wherein HIV p17 or a derivative thereof is expressed as a mature protein.

36. The method of claim 33 wherein HIV p17 is expressed as a fusion protein with a protein from the env or pol region of HIV.

37. The method of claim 33 wherein HIV p17 is expressed as a fusion protein with the S-protein of hepatitis B virus.

38. A method for producing HIV p17 protein in yeast which comprises culturing the organism transformed with an HIV p17 expression vector under permissive conditions such that the protein is expressed in recoverable quantities, and isolating the protein from the culture system.

39. The method of claim 38 wherein the organism is the yeast strain S. cerevisiae.

40. The method of claim 39 wherein the expression vector is plasmid pRIT13017 or derivatives thereof.

41. The method of claim 38 wherein HIV p17 or a derivative thereof is expressed as a mature protein.

42. The method of claim 38 wherein HIV p17 is expressed as a fusion protein with a protein from the env or pol region of HIV.

43. The method of claim 38 wherein HIV p17 is expressed as a fusion protein with the S-protein of hepatitis B virus.

44. A vaccine for stimulating protection in an individual against infection by HIV which comprises one or more recombinant HIV antigens and a suitable carrier thereof.

45. The vaccine of claim 44 wherein one of the HIV-antigens is a recombinant HIV p17 protein derived from E. coli.

46. The vaccine of claim 44 wherein one of the HIV antigens is a recombinant HIV p17 protein derived from yeast.